(19) Europäisches Patentamt
European Patent Office
Office européen des brevets

(11) **EP 2 432 787 B1**

(12) **FASCICULE DE BREVET EUROPEEN**

(45) Date de publication et mention de la délivrance du brevet:
**26.03.2014 Bulletin 2014/13**

(21) Numéro de dépôt: **10723018.7**

(22) Date de dépôt: **18.05.2010**

(51) Int Cl.:
***C07F 9/11*** *(2006.01)*

(86) Numéro de dépôt international:
**PCT/EP2010/056836**

(87) Numéro de publication internationale:
**WO 2010/133608 (25.11.2010 Gazette 2010/47)**

(54) **PROCÉDÉ DE SYNTHÈSE D'UN ORGANOPHOSPHATE DE TERRE RARE ET SON UTILISATION POUR LA PRÉPARATION D'UN SYSTÈME CATALYTIQUE "PRÉFORMÉ"**

VERFAHREN ZUR SYNTHESE EINES ORGANOPHOSPHATS EINER SELTENEN ERDE UND ANWENDUNG DAVON ZUR HERSTELLUNG EINES VORGEBILDETEN KATALYTISCHEN SYSTEMS

METHOD FOR SYNTHESISING A RARE EARTH ORGANOPHOSPHATE AND USE THEREOF TO PREPARE A "PREFORMED" CATALYTIC SYSTEM

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **19.05.2009 FR 0902426**

(43) Date de publication de la demande:
**28.03.2012 Bulletin 2012/13**

(73) Titulaires:
• **COMPAGNIE GENERALE DES ETABLISSEMENTS MICHELIN**
**63000 Clermont-Ferrand (FR)**
• **MICHELIN Recherche et Technique S.A.**
**1763 Granges-Paccot (CH)**

(72) Inventeurs:
• **THUILLIEZ, Julien**
**63040 CLERMONT-FERRAND Cedex 9 (FR)**
• **KIENER, Pierre**
**F-63360 Gerzat (FR)**
• **GUERIN, Elodie**
**F-63720 Saint-Ignat (FR)**

(74) Mandataire: **Le Cam, Véronique Marie Christine et al**
**Manufacture Française des Pneumatiques Michelin**
**23 Place des Carmes-Déchaux**
**SGD/LG/PI - F35 - Ladoux**
**63040 Clermont-Ferrand Cedex (FR)**

(56) Documents cités:
**EP-A2- 0 924 214       WO-A1-2009/019100**
**WO-A1-2009/083480**

• **YURTOV E V ET AL: "Colloid structures formed in extraction systems with organophosphorus extractants" INTERNATIONAL SOLVENT EXTRACTION CONFERENCE,, 17 mars 2002 (2002-03-17), pages 197-199, XP008118115 ISBN: 978-1-919783-25-3**

## Description

L'invention concerne un procédé de préparation d'un organophosphate de terre rare sous forme d'une solution utilisable telle quel dans un système catalytique destiné notamment à la polymérisation stéréospécifique 1,4-cis des diènes conjugués.

Dans les années 1980, les premiers systèmes catalytiques à base de terre rare ont vu le jour et leur intérêt a été grandissant, d'une part en raison de la non-toxicité du métal et d'autre part en raison du fait que des solvants non-aromatiques, tels que l'hexane, le cyclohexane, les coupes d'isomères de l'hexane ou le méthylcyclohexane sont utilisables comme solvant de polymérisation. Plus particulièrement, la catalyse au néodyme connaît actuellement un véritable essor.

Les catalyseurs de terre rare sont multi-composants et sont généralement constitués d'au moins :

- un monomère diène conjugué,
- un sel d'un ou plusieurs métaux de terre rare d'un acide phosphorique organique ou d'un acide carboxylique organique,
- un agent d'alkylation constitué d'un alkylaluminium répondant à la formule $AlR_3$ ou $HAlR_2$, et
- un donneur d'halogène constitué d'un halogénure d'alkylaluminium,

Ce type de catalyseur est notamment décrit dans les demandes de brevet internationales WO 02/38635, WO02/38636 et WO 03/097708 au nom des Demanderesses.

Parmi les sels de terre rare, les sels appelés phosphates de terre rare et de formule générale $Ln[OP(=O)_x(OR)_{3-x}]_3$ (Ln étant un métal de la famille des lanthanides, le scandium ou l'yttrium, x étant égal à 1 ou 2, R étant des radicaux alkyle aliphatiques ou alicycliques, comprenant 1 à 20 atomes de carbone, linéaires ou ramifiés, identiques ou différents) sont des composés significatifs pour la préparation de systèmes catalytiques très actifs pour la polymérisation stéréospécifique 1,4-cis des diènes conjugués, plus particulièrement le butadiène et l'isoprène. Ces systèmes présentent l'avantage de conduire à des élastomères ayant une distribution de masse molaire étroite.

De par leur nature, ces sels sont peu solubles et ont une forte tendance à s'associer en milieu solvant hydrocarboné et conduisent généralement à la formation de gel, comme décrit par Suglobov dans le document "DEHP Complexes of Lanthanides (III) and Actinides (III)", Journal of Alloys and Compounds, 213/214, 1994, p 523-527). Cela entraîne des difficultés pratiques, que ce soit lors de leur synthèse ou de leur utilisation comme précurseur de catalyseur de polymérisation.

Plusieurs méthodes de synthèse sont décrites dans la littérature. La plupart consiste en la réaction d'un sel de terres rares de type halogénure ou nitrate de terres rares avec un sel organophosphate (Korovin et al., Russian Journal of Inorganic Chemistry, 22(5), 1977 ; Chen Tien et al. "Préparation and characterization of bis(2-ethylhexyl) phosphate(P204) rare earth", K'o Hsueh T'ung Pao 1981, 26(13), 794-6 ; JP 60023406 au nom de Asahi, page 4 ).

Le document de brevet EP 1 509 557 B1 au nom des Demanderesses décrit la synthèse d'une poudre de tris(organophosphate) de néodyme $[(RO)_2P(=O)O]_3Nd$ par réaction de :

a) une solution aqueuse de chlorure de néodyme $NdCl_3(H_2O)_6$, préparé par réaction d'HCl avec du $Nd_2O_3$ , et

b) une solution eau/acétone de di(2-éthyl-hexyl)phosphate de sodium.

Ce procédé de synthèse en plusieurs étapes permet une réaction totale et conduit à la formation du sel tris[di(2-éthyl-hexyl phosphate)] de néodyme sous la forme d'un solide. Après un certain nombre de lavage de ce solide par de l'eau, le test qualitatif de détermination des ions chlorures est quasi-négatif, ce qui traduit l'obtention d'un produit quasi pur. Une fois sec, le produit se présente sous la forme d'une poudre rose-mauve.

Le document de brevet US 6,767,927 B1 décrit un procédé de synthèse en trois étapes d'une solution stable de tris(organophosphate) de terre rare dans un solvant hydrocarboné :

1) préparation d'un sel organophosphate par réaction de l'acide correspondant et d'une base,

2) réaction de l'organophosphate obtenu précédemment avec un sel de terre rare,

3) ajustement de la concentration en acide libre.

Cette synthèse est réalisée en milieu biphasique eau/solvant hydrocarboné. En fin de réaction, l'eau est éliminée par décantation et distillation azéotropique. Le produit de ce procédé de synthèse est une solution fluide utilisable directement pour la préparation de catalyseur de polymérisation. Néanmoins, le procédé comporte un certain nombre d'étapes de synthèse pour aboutir à la solution recherchée. Ce procédé génère par ailleurs des effluents aqueux contenant des ions, par exemple du nitrate d'ammonium, ce qui du point de vue environnemental nécessite une étape supplémentaire de traitement ultérieur de ces rejets.

Les procédés de synthèse précédents peuvent être définis comme étant des procédés par voie indirecte. C'est-à-dire que la synthèse du sel de phosphate de terre rare s'effectue à partir d'un sel de terre rare, obtenu par réaction de l'oxyde de terre rare et d'un acide, et d'un sel organophosphate, obtenu par réaction de l'acide correspondant et d'une base. Le nombre d'étapes fait généralement de ce type de procédé des voies de synthèse complexes et peu économiques. Un inconvénient de ces procédés est qu'ils génèrent des rejets aqueux, ce qui nécessite d'introduire une étape supplémentaire de traitement de ces rejets.

Les tris(carboxylates) de terres rares, comme par exemple les versatates de terres rares, sont des composés eux aussi utilisables comme précurseur dans la synthèse de catalyseurs pour la polymérisation stéréospécifique 1,4-cis. Leur synthèse est possible selon la même voie que précédemment, c'est-à-dire par réaction d'un sel de carboxylate avec un sel de terre rare (par exemple, réaction du versatate de sodium avec du nitrate de néodyme tel que décrit dans le document de brevet US 6,111,082).

Des voies de synthèses alternatives ont été développées pour la synthèse de sels organométalliques de cette famille afin de diminuer le coût de la synthèse en limitant le nombre d'étapes. Dans ces synthèses, le sel carboxylate ou le sel de terre rare est remplacé par leur précurseur, c'est-à-dire l'acide carboxylique correspondant ou l'oxyde de terre rare correspondant.

Le document de brevet US 6,482,906 B1 décrit un procédé de préparation de tris(néodécanoate) de néodyme, encore appelé tris(versatate) de néodyme, en quatre étapes :

1) préparation d'une boue par dispersion de $Nd_2O_3$ dans un solvant hydrocarboné et réaction avec un défaut d'HCl par rapport au néodyme,
2) ajout d'acide néodécanoïque à hauteur de 3,25 équivalents par rapport au néodyme,
3) décantation du milieu réactionnel,
4) séparation du tris(néodécanoate) de néodyme des réactifs résiduels.

Cette voie de synthèse présente l'intérêt de ne pas comporter d'étape de synthèse du sel néodécanoate par réaction avec une base, ce qui supprime une étape. L'inconvénient est que la réaction n'est pas totale et qu'une étape de séparation de l'oxyde de néodyme n'ayant pas réagi de la solution de tris(néodécanoate) de néodyme est nécessaire en vue d'utiliser le tris(néodécanoate) de néodyme pour d'autres applications, notamment pour la préparation d'un système catalytique.

Le document de brevet US 5,220,045 décrit un procédé de synthèse du néodécanoate de néodyme en une étape comprenant la réaction en milieu biphasique d'une solution aqueuse de nitrate de néodyme avec une solution organique d'acide versatique en présence d'une base de type amine, ammoniaque ou hydroxyde d'ammonium quaternaire. Lors de cette synthèse, le sel néodécanoate d'ammonium est généré in situ afin de réagir avec le nitrate de néodyme. Cette voie de synthèse présente l'intérêt de s'effectuer dans un seul réacteur et d'être totale. Elle présente l'inconvénient de nécessiter l'ajout d'une base dans le milieu réactionnel et de faire intervenir un certain nombre de lavages à l'eau et d'étapes de décantation pour débarrasser le gel de ses impuretés, ce qui est pénalisant d'un point de vue économique. Par ailleurs, Ce procédé génère des effluents aqueux contenant des ions, par exemple du nitrate d'ammonium, ce qui du point de vue environnemental nécessite une étape supplémentaire de traitement ultérieur de ces rejets.

A la différence des voies de synthèse décrites ci-dessus, une synthèse directe consiste à faire réagir directement l'oxyde de terre rare sur l'acide phosphorique ou carboxylique, sans étape intermédiaire de formation du sel de terre rare et/ou du sel de phosphate ou de carboxylate.

Le brevet EP 0 924 214 B1 concerne un procédé de fluidification des solutions organiques de composés de terres rares, tels que les phosphates ou les carboxylates de terre rares, par ajout d'acide de Lewis. L'exemple 1 de ce brevet décrit la préparation du di(2-éthyl-hexyl)phosphate de lanthane par réaction de l'oxyde de lanthane $La_2O_3$ avec l'acide di(2-éthyl-hexyl)phosphorique dans de l'hexane et une faible quantité d'eau. Le mélange est agité et porté au reflux jusqu'à obtention d'une solution jaune limpide. L'eau est ensuite éliminée par distillation azéotropique. Le gel obtenu est très visqueux, les viscosités mesurées étant supérieures à 90 000 cP et de structure fragile. Il est en effet précisé dans ce brevet qu'il y a apparition de cavités (zones de rupture) dans le gel lors de la mesure de la viscosité. Cette caractéristique gélatineuse et « cassante » rend difficile la manipulation d'un tel produit, notamment lors des étapes de mise en oeuvre pour la préparation d'un système catalytique pour la polymérisation stéréospécifique 1,4-cis des diènes conjugués, et empêche sa caractérisation. Cela implique de prendre des précautions de manipulation et de caractérisation de ce produit, notamment lors de l'utilisation de ce gel dans un procédé industriel.

La synthèse directe d'un organophosphate de terre rare en solution dans un solvant organique à partir d'un acide organophosphoré et d'un composé de terre rare est également décrite dans le document WO2009/019100. Le procédé de synthèse de cet organophosphate a la particularité d'utiliser un promoteur choisi parmi l'eau ou certains acides, et permet de préparer une solution d'organophosphate de terre rare à faible teneur en solide résiduel et avec une acidité résiduelle acceptable. Néanmoins la solution d'organophosphate préparée selon ce procédé revendiqué peut présenter une viscosité inadaptée et il peut s'avérer nécessaire d'ajuster sa viscosité par l'ajout d'un alcool, d'un acide carboxylique ou d'un acide phosphorique.

Le but de la présente invention est de remédier aux inconvénients rencontrés avec les procédés de synthèse de l'art antérieur. Plus particulièrement, un objectif de la présente invention est de proposer un procédé de synthèse d'un organophosphate de terre rare comportant un minimum d'étapes ce qui doit faciliter la mise en oeuvre à l'échelle industrielle et dont le produit soit exempt de précurseur de terre rare résiduel. Un autre objectif de l'invention est d'obtenir des solutions d'organophosphate de terre rare dont la consistance est particulièrement adaptée à une mise en oeuvre avec du matériel adapté aux produits de faible viscosité. Un autre objectif de l'invention est une synthèse d'un organophosphate de terre rare qui soit directement et aisément utilisable pour une application ultérieure, notamment pour la préparation d'un système catalytique. Un autre objectif de l'invention est de mettre en oeuvre un procédé ne générant très peu d'effluents aqueux nécessitant un traitement ultérieur afin de répondre à une problématique environnementale.

Ce but est atteint en ce que les inventeurs viennent de découvrir, au cours de leurs recherches, un procédé de synthèse par voie directe d'un organophosphate de terre rare dont le produit est, de manière inattendue, exempt d'oxyde de terre rare résiduel ou de toute impureté, dans des quantités pénalisant toute utilisation ultérieure. Le procédé de synthèse par voie directe conduit à un produit dont la manipulation n'est pénalisée ni par un caractère « cassant », ni par une viscosité élevée qui gêneraient sa mise en oeuvre avec un matériel standard et son utilisation lors de la préparation d'un système catalytique pour la polymérisation 1,4-cis des diènes conjugués. L'organophosphate de terre rare obtenu par ce procédé se présente sous forme de solution très peu visqueuse ou fluide, même lorsque la teneur massique en néodyme est élevée (par exemple jusqu'à 8%). De manière tout aussi inattendue, il s'avère que cette solution est utilisable aisément, telle quel notamment comme précurseur d'un système catalytique destiné à la polymérisation stéréospécifique 1,4-cis des diènes conjugués sans altération de l'activité catalytique du système ou des caractéristiques du polymère synthétisé. Le procédé de synthèse selon l'invention ne génère pas d'effluents aqueux nécessitant un traitement ultérieur.

L'invention a donc pour objet un procédé de synthèse par voie directe d'un organophosphate de terre rare à partir de l'oxyde de terre rare, d'un acide organophosphorique et d'une faible quantité d'acide organique ou minéral, en une seule étape.

Le procédé de synthèse de l'invention permettant de préparer un organophosphate de terre rare est un procédé de synthèse par voie directe qui comprend :

- la dispersion sous fort cisaillement d'un oxyde de terre rare dans un milieu comprenant au moins un solvant organique, de l'eau, un acide organophosphorique et une faible quantité d'un acide organique ou minéral, et simultanément ou séquentiellement ;

- la réaction de l'oxyde de terre rare avec l'acide organophosphorique et la faible quantité d'acide organique ou minéral.

La dispersion sous fort cisaillement selon l'invention peut être réalisée par tout moyen approprié à l'aide de tout mélangeur dynamique connu de l'homme de l'art. Conviennent particulièrement les dispositifs constitués d'un agitateur rotatif comprenant un ou plusieurs mobiles fixés sur un arbre, qui dissipent des puissances volumiques de l'ordre de 0,1 à 250 kW/m3/unité de viscosité dynamique du mélange réactionnel, plus particulièrement de l'ordre de 1 à 150 kW/m3/Pa.s. Le cisaillement dépend des caractéristiques du mobile, en particulier de sa géométrie. Des dispositifs de ce genre sont par exemple des mélangeurs à rotor et stator, notamment ceux commercialisés par la société VMI Rayneri sous la marque "Ultramix" et par la société IKA sous la marque "Ultra Turrax".

Par " fort cisaillement " on doit comprendre un " taux de cisaillement élevé " ou un " gradient de vitesse élevé ", qui sont deux expressions synonymes pour l'homme de l'art. Par le terme "fort" ou "élevé" on entend dans le cas présent suffisamment fort ou élevé pour atteindre les propriétés recherchées des organophosphates de terre rare préparés selon le procédé de l'invention.

La notion de "gradient de vitesse moyen" dans une cuve agitée est définie par exemple dans l'article " Agitation Mélange - concepts théoriques de base ", écrits par Michel Roustan, Jean-Claude Pharamond, Alain Liné de l'ouvrage " Techniques de l'ingénieur, traité Génie des Procédés - J3 800, page 13". Cette notion est par exemple utilisée dans le brevet US 6,638,918, qui la décrit de la même manière que dans la référence précédente. Ainsi, on peut exprimer le cisaillement moyen ($\Gamma_{moyen}$) dans le milieu par la formule :

$$\Gamma_{moyen} = k \ N$$

dans laquelle k est une constant de proportionnalité qui dépend du type de pale du mélangeur et de la configuration de la cuve du mélangeur et N est la vitesse des mobiles d'agitation (en $s^{-1}$) . $\Gamma_{moyen}$ est exprimé en $s^{-1}$. Les mélangeurs

utilisables pour mettre en oeuvre le procédé selon l'invention présente une constante k variant entre 10 et 13.

On associe à cette notion de cisaillement une vitesse périphérique en bout de pale, exprimée par l'expression :

$$V_{périph} = \pi \ N \ D$$

dans laquelle D est le diamètre des mobiles d'agitation exprimé en mètre et N leur vitesse de rotation exprimée en $s^{-1}$.

Selon l'invention, on entend par "dispersion sous fort cisaillement", une dispersion effectuée sous une agitation à grande vitesse en bout de pale, typiquement supérieure à 4 $m.s^{-1}$, de préférence supérieure ou égale à 10 $m.s^{-1}$, et encore plus préférentiellement comprise entre 10 $m.s^{-1}$ et 50 $m.s^{-1}$, qui impartit un cisaillement au milieu, typiquement supérieur à 200 $s^{-1}$, de préférence compris entre 200 $s^{-1}$ et 50000 $s^{-1}$, plus préférentiellement encore entre 200 $s^{-1}$ et 20000 $s^{-1}$.

En fonction du niveau de cisaillement recherché, l'homme du métier saura utiliser un système d'agitation ayant une géométrie adaptée et le mettre en oeuvre à une vitesse de rotation suffisante pour atteindre la vitesse de rotation en bout de pales et le niveau de cisaillement recherchés. Selon la nature du système d'agitation et la taille du réacteur de synthèse, la vitesse de rotation peut à titre d'exemple être comprise entre 100 $tr.min^{-1}$ et 30 000 $tr.min^{-1}$.

La température du milieu pendant la dispersion sous fort cisaillement de l'oxyde de terre rare dans ce milieu et la réaction de l'oxyde de terre rare avec l'acide organophosphorique et l'acide organique ou minéral est définie en fonction de la nature des solvants utilisés et de la quantité totale d'acide phosphorique introduite dans le milieu. La température du milieu est généralement comprise entre 0 et 130°C. Cette température est préférentiellement comprise entre 20 et 110°C, plus préférentiellement encore entre 20 et 90°C.

La durée de la dispersion de l'oxyde de terre rare dans le milieu peut varier de 1 à 100 minutes, préférentiellement elle est de 1 à 60 minutes. La durée globale de la synthèse comprenant notamment l'étape de dispersion de l'oxyde de terre rare et de l'étape de réaction de l'oxyde de terre rare avec l'acide organophosphorique et l'acide organique ou minéral est de 1 à 600 minutes.

Selon l'invention, on entend par "terre rare" un métal choisi parmi l'yttrium, le scandium et les lanthanides, métaux ayant un numéro atomique allant de 57 à 71 inclus dans le tableau périodique des éléments de Mendeleïev. De préférence le métal de terre rare est choisi parmi les lanthanides tels que notamment le lanthane, le praséodyme, le gadolinium ou le néodyme, le néodyme étant plus particulièrement préféré.

Bien que le procédé de l'invention s'applique également à l'yttrium et au scandium, l'invention sera décrite dans le paragraphe qui suit par référence aux lanthanides pour des raisons de simplification de l'écriture.

L'un des réactifs du procédé de l'invention est l'oxyde de lanthanide. Selon l'invention, on entend par oxyde de lanthanide toute espèce de type sesquioxyde de lanthanide répondant à la formule $Ln_2O_3$, mais aussi toute forme hydratée, partiellement ou en totalité, d'oxyde de lanthanide contenant des fonctions Ln-OH. En effet, de par leur caractère hygroscopique, les oxydes de lanthanide peuvent absorber une certaine quantité d'eau sous l'effet de l'humidité de l'air ambiant. Cette eau peut conduire à l'hydratation de l'oxyde de lanthanide et à la formation d'autres espèces à base de lanthanides, tel que le tris(hydroxyle) de lanthanide, selon l'équation :

$$Ln_2O_3 + 3 \ H_2O \rightleftarrows 2 \ Ln(=O)OH + 2 \ H_2O \rightleftarrows 2 \ Ln \ (OH)_3$$

A titre d'illustration dans le cas où le métal de lanthanide est le néodyme, le taux massique en néodyme théorique du sesquioxyde de néodyme, noté $\%Nd[Nd_2O_3]_{théo}$ est de 85,74 %. Le taux massique en néodyme théorique du tris(hydroxyle) de néodyme, noté $\%Nd[Nd(OH)_3]_{théo}$ est de 73,87 %.

Le taux massique de néodyme de la matière première utilisée, noté $\%Nd[Nd_2O_3]$ est déterminée selon toute méthode de caractérisation connue de l'homme du métier. Par exemple, peuvent être utilisées les méthodes de dosage décrites dans la demande de brevet WO03/097708 au nom des Demanderesses.

On définit ainsi le degré d'hydratation $D_{hydratation}$ d'un oxyde de lanthanide comme étant :

$$D_{Hydratation} = \frac{\%Nd[Nd_2O_3] - \%Nd[Nd(OH)_3]_{théo}}{\%Nd[Nd_2O_3]théo - \%Nd[Nd(OH)_3]_{théo}}$$

Ainsi, le degré d'hydratation $D_{hydratation}$ selon l'invention de sesquioxyde de néodyme pur de formule $Nd_2O_3$ est de 0%, tandis que le degré d'hydratation de tris(hydroxyle) de néodyme de formule $Nd(OH)_3$ est de 100%.

Préférentiellement, le procédé de synthèse selon l'invention met en jeu un oxyde de terre rare ayant un degré d'hydratation allant de 0 à 100%, préférentiellement compris entre 0 et 50% et encore plus préférentiellement compris entre 0 et 20%.

L'acide organophosphorique, deuxième réactif du procédé selon l'invention, est choisi parmi les composés suivants: les diesters d'acide phosphorique de formule générale (RO)(R'O)PO(OH), dans laquelle R est un radical alkyle, aryle ou alkylaryle et R' est un radical alkyle, aryle ou alkylaryle; les monoesters d'acide phosphorique de formule générale $(RO)PO(OH)_2$ dans laquelle R est un radical alkyle, aryle ou alkylaryle; les phosphonates de formule générale (RO)R'P(O) ou $RP(O)(OH)_2$ dans lesquelles R est un radical alkyle, aryle ou alkylaryle; les phosphinates de formule générale R(R')P(O)OH ou R(H)P(O)OH dans lesquelles R est un radical alkyle, aryle ou alkylaryle; et leurs mélanges.

De préférence, l'acide organophosphorique est choisi parmi les diesters d'acide phosphorique de formule générale (RO)(R'O)PO(OH), dans laquelle R est un radical n-butyle, isobutyle, pentyle, amyle, isopentyle, 2,2-diméthylhexyle, 1-éthylhexyle, 2-éthylhexyle, tolyle, nonaphenoxyle et R'est un radical n-butyle, isobutyle, pentyle, amyle, isopentyle, 2,2-diméthylhexyle, 2- éthylhexyle, 1-éthylhexyle, tolyle, nonaphenoxyle; les monoesters d'acide phosphorique de formule générale $(RO)PO(OH)_2$ dans laquelle R est un radical n-butyle, isobutyle, pentyle, amyle, isopentyle, 2,2-diméthylhexyle, 1-éthylhexyle, 2- éthylhexyle, tolyle, nonaphenoxyle; les phosphonates de formule générale (RO)R'P(O) ou $RP(O)$ $(OH)_2$ dans lesquelles R est un radical n-butyle, isobutyle, pentyle, amyle, isopentyle, 2,2-diméthylhexyle, 2-éthylhexyle, 1-éthylhexyle, tolyle, nonaphenoxyle; les phosphinates de formule générale R(R')P(O)OH ou R(H)P(O)OH dans lesquelles R est un radical n-butyle, isobutyle, pentyle, amyle, isopentyle, 2,2-diméthylhexyle, 2- éthylhexyle, 1-éthylhexyle, tolyle, nonaphenoxyle; et leurs mélanges. A titre encore plus préférentiel, l'acide organophosphorique selon l'invention est choisi parmi l'acide di(2-éthylhexyl)phosphorique.

Le rapport molaire de l'acide organophosphorique à la terre rare est fonction de la nature de l'acide organophosphorique. L'homme du métier ajustera la quantité d'acide organophosphorique à introduire dans le milieu en fonction de sa nature.

Lorsque l'acide organophosphorique est un diester d'acide phosphorique, le rapport molaire de l'acide organophosphorique à la terre rare est 2,7 à 4, de préférence de 2,75 à 3,5, et encore plus préférentiellement de 2,8 à 3,3.

L'acide organique ou minéral, troisième réactif du procédé selon l'invention est choisi parmi les acides organiques ou minéraux de pKa à 25°C inférieur à 2,5, de préférence allant de -10 à 2,5 , encore plus préférentiellement -1 à 2, et leur mélanges.

L'acide minéral ou organique est choisi parmi les composés suivants : l'acide perchlorique $HClO_4$, l'acide chlorique $HClO_3$, l'acide chloreux $HClO_2$, l'acide chlorhydrique HCl, l'acide bromique $HBrO_3$, l'acide bromhydrique HBr, l'acide iodique $HIO_3$, l'acide periodique $HIO_4$, l'acide orthoperiodique $H_5IO_6$, l'acide iodhydrique HI, l'acide trichloroacé-tique $CCl_3$-COOH, l'acide dichloroacétique $CCl_2$H-COOH, l'acide trifluoroacétique $CF_3COOH$, l'acide nitrique $HNO_3$, l'acide peroxonitrique $HNO_4$, l'acide sulfureux $H_2SO_3$, l'acide sulfurique $H_2SO_4$, les hydrogénosulfates de lithium, de sodium ou de potassium, l'acide fluorosulfurique $FSO_3H$, l'acide sulfonique $HSO_3H$, l'acide trifluorométhanesulfonique $CF_3$-$SO_3H$, l'acide benzène-sulfonique, l'acide naphtalène-sulfonique, l'acide phosphorique $H_3PO_4$, les hydrogénophos-phates de lithium, de sodium ou de potassium, l'acide pyrophosphorique $H_4P_2O_7$, l'acide phosphoreux $H_3PO_3$, l'acide hypophosphoreux $H_3PO_2$, l'acide chromique $H_2CrO_4$, l'acide picrique, l'acide maléique, l'acide oxalique et leurs mélanges.

Encore plus préférentiellement, l'acide minéral ou organique est choisi par mi les composés suivants : l'acide chlorhydrique HCl, l'acide trichloroacétique $CCl_3$-COOH, l'acide trifluoroacétique $CF_3COOH$, l'acide nitrique $HNO_3$, l'aci-de sulfurique $H_2SO_4$, les hydrogénosulfates de lithium, de sodium ou de potassium, l'acide trifluorométhanesulfonique $CF_3$-$SO_3H$, l'acide phosphoreux $H_3PO_3$ ou l'acide hypophosphoreux $H_3PO_2$ et leurs mélanges. Selon une variante particulièrement préférée de l'invention, on choisit parmi les acides minéraux, l'acide chlorhydrique ou l'acide nitrique.

De manière à ne pas pénaliser l'utilisation ultérieure de la solution d'orgaphosphate de terre rare dans un catalyseur pour la polymérisation des diènes conjugués, on utilise dans le procédé selon l'invention une faible quantité d'acide organique ou minéral. Par faible quantité, on entend selon l'invention une quantité telle que le rapport molaire de l'acide organique ou minéral par rapport à la terre rare est compris entre 0 et 0,25 équivalents (les bornes de cet intervalle étant exclues), préférentiellement le rapport molaire a une valeur allant de 0,03 à 0,20 équivalents (les bornes étant incluses).

Le rapport molaire de la quantité totale d'acides dans le milieu à la terre rare est fonction de la nature de l'acide organophosphorique et de l'acide minéral ou organique. Par quantité totale d'acides dans le milieu, on entend la somme de la quantité d'acide organophosphorique et d'acide minéral ou organique selon l'invention. L'homme du métier ajustera la quantité à introduire dans le milieu en fonction de la nature des acides présents dans le milieu. Ainsi, avantageusement, la quantité totale d'acides dans le milieu est choisie telle que le rapport molaire de la quantité totale d'acides par rapport à la terre rare est compris entre 2,70 et 4,25. De préférence ce rapport molaire est compris entre 2,78 et 3,68 et encore plus préférentiellement entre 2,85 et 3,45.

Le rapport molaire de l'acide organique ou minéral à la terre rare est fonction de sa nature, ainsi que de la nature de l'acide organophosphorique et du rapport molaire ce dernier par rapport à la terre rare. L'homme du métier ajustera cette quantité d'acide organique ou minéral à introduire dans le milieu en fonction de la nature et de la quantité des réactifs choisies.

L'acide organique ou minéral selon l'invention est introduit dans le milieu réaction sous forme pur ou sous forme d'une solution aqueuse. Dans ce dernier cas, l'acide organique ou minéral selon l'invention est introduit soit sous forme d'une solution aqueuse concentrée, soit diluée dans la quantité d'eau introduite en tout début de synthèse.

L'acide organique ou minéral selon l'invention est introduit seul ou en mélange avec un ou plusieurs autres acides organiques ou minéraux selon l'invention.

Selon le procédé de l'invention, le milieu comprend au moins un solvant organique. On entend par solvant organique dans le cadre de l'invention un ou plusieurs solvants comprenant au moins un solvant hydrocarboné inerte. Selon un mode préférentiel de réalisation de l'invention, le solvant hydrocarboné inerte est un solvant aliphatique et alicyclique de bas poids moléculaire, tel que, à titre indicatif, le n-pentane, l'iso-pentane, le cyclopentane, une coupe d'isomères du pentane, une coupe de composés en C5, une coupe C5 de vapocraquage de naphta, l'hexane, le cyclohexane, une coupe d'isomères de l'hexane, le méthylcyclohexane, l'heptane, leurs isomères ou un mélange de ces solvants. A titre encore plus préférentiel, on utilise le n-pentane, une coupe de composés en C5, le cyclohexane, le méthylcyclohexane ou leur mélange à titre de solvants hydrocarbonés inertes.

Selon une mise en oeuvre préférentielle de l'invention, le rapport en volume de l'eau par rapport au solvant hydrocarboné inerte est strictement supérieur à 0 et strictement inférieur à 1.

Selon une variante du procédé de l'invention, le milieu peut également comprendre d'autres composants susceptibles d'agir sur la mise en oeuvre du procédé ou encore sur les propriétés du produit obtenu

Selon une variante du procédé de synthèse de l'invention, les étapes de dispersion sous fort cisaillement de l'oxyde de terre rare dans le milieu et de réaction de l'oxyde de terre rare avec l'acide organophosphorique et l'acide organique ou minéral peuvent être mise en oeuvre dans un même réacteur (synthèse "one-pot"). Dans ce cas, la dispersion de l'oxyde de terre rare dans le milieu et la réaction de l'oxyde de terre rare avec l'acide organophosphorique et l'acide organique ou minéral peuvent avoir lieu simultanément ou séquentiellement. Dans ce cas, l'agitation sous fort cisaillement est activée uniquement pendant la durée nécessaire à la dispersion.

Selon une autre variante du procédé de synthèse de l'invention, les étapes de dispersion sous fort cisaillement de l'oxyde de terre rare dans le milieu et de réaction de l'oxyde de terre rare avec l'acide organophosphorique et l'acide organique ou minéral peuvent être mises en oeuvre dans deux réacteurs différents. Après la dispersion sous fort cisaillement de l'oxyde de terre rare dans le milieu, le mélange est transféré dans un deuxième réacteur. Ce deuxième réacteur peut être équipé d'un système d'agitation mécanique classique à pales et la réaction s'effectue sous une agitation mécanique modérée. Le milieu dans les deux réacteurs est généralement chauffé à une température de 0 et 100°C pendant une durée globale du procédé variant de 1 et 400 minutes. Selon cette variante, les étapes du procédé de l'invention peuvent se dérouler séquentiellement ou simultanément, la réaction de l'oxyde de terre rare avec l'acide organophosphorique et l'acide organique ou minéral pouvant par exemple avoir débuté durant la dispersion sous fort cisaillement de l'oxyde de terre rare dans le milieu, dans le premier réacteur, et être poursuivie dans le deuxième réacteur.

Après réaction de l'acide phosphorique et l'acide organique ou minéral avec l'oxyde de terre rare, la solution d'organophosphate de terre rare obtenue comprend de l'eau et doit donc être séchée, notamment en vue de l'utilisation ultérieure de la solution d'organophosphate de terre rare pour la préparation d'un catalyseur de polymérisation des diènes conjugués. Cette élimination peut être effectuée par tout procédé connu de l'homme du métier. Selon une variante préférentielle de l'invention, l'eau est éliminée par distillation azéotropique.

La mise en oeuvre du procédé de l'invention permet la synthèse d'un organophosphate de terre rare sous forme d'une solution très peu visqueuse ou fluide avec un taux élevé de conversion de l'oxyde de terre rare. Aucune étape de lavage ou de purification supplémentaire n'est nécessaire. Ceci n'exclut toutefois pas de telles étapes tout en restant dans le cadre de l'invention. Lorsqu'une telle étape de lavage ou de purification supplémentaire est réalisée, la réalisation d'un traitement ultérieur des rejets aqueux n'est pas nécessaire, ceux-ci étant soient exempts de tout sous-produit de la réaction, soit recyclables dans le procédé de synthèse.

En effet, selon une autre variante du procédé de synthèse de l'invention, une étape optionnelle de lavage de la solution de phosphate de terre rare est réalisée immédiatement après la réaction de l'acide phosphorique et l'acide orgainque ou minéral sur l'oxyde de terre rare. Cette étape de lavage consiste à introduire dans le milieu, comprenant l'organophosphate de terre rare, un volume supplémentaire d'eau, puis à agiter le milieu biphasique ainsi résultant de manière à laver la solution, puis enfin à séparer par toute méthode connue de l'homme de l'art la phase aqueuse de la solution organique d'organophosphate de terre rare. Cette étape de lavage permet notamment l'élimination des réactifs en excès n'ayant pas réagi, tels que par exemple l'acide organophosphorique et les espèces ioniques hydrosolubles issues de l'acide organique ou minéral. L'homme du métier ajustera les conditions de lavage, en terme de volume d'eau utilisé, des conditions d'agitation mises en oeuvre et du nombre de lavage consécutifs afin d'atteindre les caractéristiques du produit recherchées.

Cette quantité d'eau supplémentaire doit être éliminée en fin de lavage, notamment en vue de l'utilisation ultérieure de la solution d'organophosphate de terre rare pour la préparation d'un catalyseur de polymérisation des diènes conjugués. Cette quantité d'eau peut éventuellement être recyclée en amont du procédé de synthèse, de manière à ré-utiliser l'acide organophosphorique, tel que l'acide di(2-éthyl-hexyl)phosphorique, n'ayant pas réagi.

La solution obtenue par le procédé de synthèse de l'invention comprend au moins un organophosphate de terre rare et un solvant hydrocarboné inerte tel que défini précédemment. Elle peut également comprendre un ou plusieurs acides libres résiduels, ainsi qu'une faible quantité d'eau.

Selon une variante préférentielle, le procédé de l'invention permet la synthèse d'organophosphate de lanthanide, et plus particulièrement un organophosphate de néodyme sous forme d'une solution très peu visqueuse ou fluide. La synthèse, conformément au procédé de l'invention, du tris[di(2-éthylhexyl)phosphate de néodyme constitue un aspect particulièrement préféré de cette variante.

La solution d'organophosphate de néodyme obtenue préférentiellement par la mise en oeuvre du procédé de synthèse selon l'invention possède avantageusement, avant tout traitement ultérieur, les caractéristiques suivantes :

- un taux massique de Nd est compris entre 2,2 et 8,5%, préférentiellement entre 2,3 et 8% ;

- une acidité résiduelle dans la solution comprise entre 0 et 25 mmol pour 100 g de sel et préférentiellement entre 1 et 15 mmol pour 100 g de sel;

- une viscosité comprise entre 200 et 40 000 cP, préférentiellement comprise entre 1000 et 20 000 cP.

Cette solution d'organophosphate de néodyme présente également de manière préférentielle les caractéristiques suivantes :

- une quantité d'eau résiduelle dans la solution est inférieure à 500 ppm, préférentiellement inférieure à 200 ppm et encore plus préférentiellement inférieur à 100 ppm.

- un excès molaire d'acide organophosphorique par rapport au néodyme a une valeur allant de 0 à 100%, préférentiellement de 0 à 50% et encore plus préférentiellement de 0 à 30%;

La solution d'organophosphate de terre rare obtenue par le procédé conforme à l'invention peut avantageusement être utilisée telle quel ou il peut subir des modifications après sa préparation et avant toute utilisation ultérieure, sans que cela sorte du cadre de l'invention. Dans la présente demande, on entend par modification tout traitement que pourrait subir la solution obtenue directement par le procédé de l'invention, par exemple l'élimination de tout ou partie de l'acide libre résiduel par toute méthode connue de l'homme du métier, tel que notamment le lavage par une solution aqueuse, le lavage par une solution aqueuse contenant une base, tel que de l'ammoniaque ou de la soude, le lavage par une solution aqueuse contenant un acide ou encore l'adjonction d'agents divers tels que par exemple certains dérivés de l'aluminium répondant à la formule $Al(R)_{3-n}H_n$, où n a pour valeur 0, 1 ou 2 et R est un hydrocarbure comportant 1 à 8 atomes de carbone, tel que par exemple notamment le triéthylaluminium, le tri-iso-butylaluminium, le trioctylaluminium ou encore l'hydrure de dis-isobutylaluminium.

A solution d'organophosphate de terre rare obtenue par le procédé conforme à l'invention peut être utilisée telle quel par exemple pour la préparation de systèmes catalytiques pour la polymérisation stéréospécifique de diènes conjugués. Notamment elle peut être utilisée telle quel, conjointement avec un agent d'alkylation, un alkylaluminium par exemple, et conjointement avec un donneur d'halogène, un chlorure de dialkylaluminium par exemple, sans que cela nuise à la préparation du catalyseur, à son activité ou aux caractéristiques du polymères préparés.

Ainsi, un autre objet de l'invention est la préparation d'un système catalytique "préformé" pour la polymérisation stéréospécifique de diènes conjugués à base d'au moins :

- un monomère diène conjugué,
- la solution d'organophosphate de terre rare décrite précédemment
- un agent d'alkylation constitué d'un alkylaluminium de formule $AlR_3$ ou $HAlR_2$ et
- un donneur d'halogène qui appartient à la famille des halogénures d'alkylaluminium à l'exclusion des sesquihalogénures d'alkylaluminium,

qui comprend les étapes de préparation de la solution d'organophosphate de terre rare, particulièrement le tris(organophosphate) de néodyme selon le procédé conforme à l'invention décrit plus haut.

Ce procédé est mis en oeuvre sans altération liée à la nature de l'organophosphate de terre rare et/ou à son procédé d'obtention.

Plus particulièrement, le procédé de préparation de ce système catalytique "préformé" selon l'invention met en oeuvre les étapes suivantes :

- dans une première étape on prépare la solution d'organophosphate de terre rare, plus particulièrement l'organophosphate de néodyme selon le procédé décrit plus haut,
- dans une deuxième étape on ajoute à la solution d'organophosphate de terre rare obtenue un monomère diène conjugué,
- dans une troisième étape, on ajoute un agent d'alkylation à la solution obtenue au terme de la deuxième étape pour l'obtention d'un sel alkylé, et
- dans une quatrième étape, on ajoute un donneur d'halogène au sel alkylé obtenu précédemment.

Les monomères de diènes conjugués, les agents d'alkylation et les donneurs d'halogène utilisables dans le cadre du procédé de préparation du système catalytique de l'invention sont tels que définis notamment dans le brevet EP 1 509 557 B1 au nom des Demanderesses.

Le système catalytique selon l'invention est particulièrement adapté pour la polymérisation stéréospécifique 1,4-cis de diènes conjugués, en particulier du butadiène et de l'isoprène, en continu ou en discontinu.

Le procédé de polymérisation de diènes conjugués consiste à faire réagir au moins un monomère de diène conjugué en présence du système catalytique de l'invention dans un solvant de polymérisation hydrocarboné inerte, par exemple du cyclohexane, du méthylcyclohexane ou leur mélange. Ce procédé est de préférence mis en oeuvre à une température allant de 0° C à 100° C.

Le polymère ainsi préparé présente un taux élevé d'unités 1,4-cis, de préférence supérieur à 97%, et une macrostructure possédant une distribution de masse molaire étroite.

A titre d'exemple, on peut citer la préparation de polybutadiène par polymérisation en discontinu avec un système catalytique à base de d'organophosphate de néodyme de l'invention, présentant un indice de polydispersité Ip, mesuré par la technique "SEC", inférieur à 2, ou encore la préparation en discontinu de polyisoprène présentant un indice de polydispersité inférieur à 2,7. La polymérisation en continu avec le même système catalytique permet l'obtention de polybutadiène et de polyisoprène présentant respectivement des valeurs Ip inférieures à 2,7 et 3,5.

Les caractéristiques précitées de la présente invention, ainsi que d'autres, seront mieux comprises à la lecture de la description suivante de plusieurs exemples de réalisation de l'invention, donnés à titre illustratif et non limitatif, ainsi que du diagramme qui représente l'activité catalytique de systèmes catalytiques conformes à l'invention et de systèmes catalytiques de l'état de la technique, évaluée par mesure du taux de conversion du monomère en polymère en fonction du temps de réaction.

## 1. SYNTHESE DE SELS DE PHOSPHATE ORGANIQUE DE NEODYME

On a synthétisé du tris(di(2-éthyl-hexyl)phosphate) de néodyme. On a utilisé pour chaque essai les conditions de synthèse qui sont détaillées ci-après.

### Matières premières

#### 1-Oxyde de néodyme

On utilise un oxyde de néodyme avec un taux massique en néodyme égal à 73,6% (type A) ou un oxyde de néodyme avec un taux massique en néodyme égal à 82,4% massique (type B).

#### 2-Acides organiques ou minéraux

Les composés acides ont été obtenus auprès de Sigma-Aldrich et ont été utilisés sous la forme de solution aqueuse ayant une concentration comprise entre 3 et 5 mol/L.

#### 3-Autres réactifs

L'acide di(2-éthyl-hexyl) phosphorique, le cyclohexane, le méthylcyclohexane et les autres réactifs ont été obtenus auprès de Sigma-Aldrich, Acros Organics, Crompton, Fluka et Sigma-Aldrich.

### Techniques de caractérisation.

#### 1-Dosage du Nd et du P

Les teneurs massiques en néodyme et en phosphore sont déterminées par analyses élémentaires par complexométrie.

*2-Dosage des espèces acides libres*

La quantité d'acides libres résiduels est déterminée par dosage potentiométrique par réaction acido-basique.

*3-Dosage de l'eau*

La méthode Karl-Fisher est utilisée.

*4-Taux d'insolubles*

La quantité d'insolubles présents dans le gel ou la solution de tris(di(2-éthyl-hexyl)phosphate) de néodyme est déterminée par centrifugation du gel ou de la solution à la température de 20°C à la vitesse de 3 500 tr.min$^{-1}$ et est exprimée en pourcentage massique de solide par rapport au gel ou à la solution.

*5-Mesure de la viscosité du tris(di(2-éthyl-hexyl)phosphate) de néodyme*

L'appareillage utilisé pour déterminer la viscosité de la solution est un rhéomètre à contrainte imposé de marque "Haake", modèle "Rheostress 1". Les plateaux utilisés sont indifféremment de type plan-plan (diamètre 35 mm) ou cône-plan (diamètre 60 mm). L'entrefer entre le rotor supérieur et le rotor inférieur est typiquement de 0,5 mm. La température de mesure est de 30°C. La valeur de la viscosité est déterminée pour un taux de cisaillement de 1 s$^{-1}$. La viscosité mesurée est exprimée en cP (centipoise).

**1) Synthèse d'un sel de phosphate organique de néodyme selon le Procédé décrit dans brevet EP 0 924 921 B1 :**

Les contre-exemples qui suivent permettent de mettre en évidence l'intérêt d'une agitation sous fort cisaillement.

Contre-Exemple 1 :

Dans un réacteur de 1 Litre à double enveloppe muni d'un Dean-Stark, d'un réfrigérant, d'un système d'agitation mécanique à pales, on introduit 15,59 g de $Nd_2O_3$ de type A (0,040 mol), 83,8 g d'acide di(2-éthyl-hexyl)phosphorique (DEHPA) (0,249 mol), 560 ml de méthylcyclohexane et 20 ml d'eau. Le mélange est porté au reflux et agité pendant 300 minutes. On remarque la présence d'un solide en suspension. L'eau du milieu est ensuite éliminée par distillation azéotropique. Après sous-tirage du réacteur, on obtient 490 g d'un gel turbide visqueux et cassant et qui présente des particules solides en suspension. Le solide ne peut pas être séparé du gel par centrifugation, en raison de la trop forte viscosité du gel.
Cet essai montre que les conditions de synthèse décrites dans l'exemple 1 du brevet EP 0 924 214 B1 appliquées à l'oxyde de néodyme et à un solvant alicyclique ne permettent pas d'obtenir un gel de tris(organophosphate) limpide et exempt de résidu solide en suspension.

Contre-Exemple 2 :

Dans un réacteur de 1 Litre à double enveloppe muni d'un Dean-Stark, d'un réfrigérant, d'un système d'agitation mécanique à pales, on introduit 13,99 g de $Nd_2O_3$ de type B (0,040 mol), 83,8 g d'acide di(2-éthyl-hexyl)phosphorique (DEHPA) (0,249 mol), 560 ml de méthylcyclohexane et 20 ml d'eau. Le mélange est porté au reflux et agité pendant 300 minutes. On remarque la présence d'un solide en suspension. L'eau du milieu est ensuite éliminée par distillation azéotropique. Après sous-tirage du réacteur, on obtient 497 g d'un gel visqueux et cassant et qui présente un solide en suspension. Le solide ne peut pas être séparé du gel par centrifugation, en raison de la trop forte viscosité du gel.
Cet essai montre que les conditions de synthèse décrites dans l'exemple 1 du brevet EP 0 924 214 B1 appliquées à l'oxyde de néodyme et à un solvant alicyclique ne permettent pas d'obtenir un gel de tris(organophosphate) limpide et exempt de résidu solide en suspension.

Contre-Exemple 3 :

Dans un réacteur de 1 litre à double enveloppe muni d'un Dean-Stark, d'un réfrigérant, d'un système d'agitation mécanique à pales, on introduit 15,59 g de $Nd_2O_3$ de type A (0,040 mol), 92,3 g d'acide di(2-éthyl-hexyl)phosphorique (DEHPA) (0,275 mol), 550 ml de méthylcyclohexane et 20 ml d'eau. Le mélange est porté au reflux et agité pendant 120 minutes. On remarque la présence d'un solide en suspension. L'eau du milieu est ensuite éliminée par distillation azéotropique. Après sous-tirage du réacteur, on obtient 480 g d'un gel présentant un solide en suspension (taux massique de solide = 0,3%).

Cet essai démontre, en fluidifiant le gel obtenu pour une meilleure centrifugation grâce à l'ajout d'une quantité supplémentaire de DEHPA, que le gel contient une quantité de solide résiduel non négligeable.

L'ensemble de ces exemples démontrent que les conditions de synthèse décrites dans le document de brevet EP 0 924 214 B1 appliquée au métal néodyme et à un solvant alicyclique ne permettent pas d'obtenir une solution d'organophosphate de néodyme limpide et peu visqueuse, voire fluide.

**3) Synthèse d'un sel de phosphate organique de néodyme selon le procédé de l'invention :**

Exemple 1

Dans un bécher de 1 Litre on introduit dans on introduit 15,83 g de $Nd_2O_3$ (0,090 mol de Nd), 94,78 g d'acide di(2-éthyl-hexyl)phosphorique (DEHPA) (0,288 mol), 505 g de cyclohexane, 21 ml d'eau. Le mélange subit alors un traitement avec un rotor-stator de marque Ultra-Turrax à la vitesse de 3000 tr.min$^{-1}$, de manière à disperser l'oxyde de néodyme dans le milieu. Dès le début du traitement avec le rotor-stator sont ajoutées 0,0162 mol d'acide chlorhydrique HCl concentré, soit 0,18 équivalents par rapport au néodyme, sous la forme d'une solution aqueuse concentrée. Le traitement s'effectue pendant une durée de 20 minutes. Le milieu est ensuite transféré dans un réacteur de 1 litre à double enveloppe muni d'un Dean-Stark, d'un réfrigérant, d'un système d'agitation mécanique à pales. Le mélange est porté à ébullition et l'eau du milieu est éliminée par distillation azéotropique. Après soutirage du réacteur, on obtient une solution violette translucide et fluide dont les caractéristiques sont présentées dans le tableau 1.

Exemple 2

Le mode opératoire est le même que dans l'exemple 1, sauf que la quantité d'acide chlorhydrique est remplacée par 0,054 mol d'acide nitrique $HNO_3$ concentré, soit 0,06 équivalents par rapport au néodyme. On obtient une solution violette translucide et fluide dont les caractéristiques sont présentées dans le tableau 1.

Exemple 3

Dans un bécher de 1 Litre on introduit dans on introduit 57,07 g de $Nd_2O_3$ (0,324 mol de Nd), 318,57 g d'acide di(2-éthyl-hexyl)phosphorique (DEHPA) (0,968 mol), 241 g de cyclohexane, 10 ml d'eau. Le mélange subit alors un traitement avec un rotor-stator de marque Ultra-Turrax à la vitesse de 3000 tr.min$^{-1}$, de manière à disperser l'oxyde de néodyme dans le milieu. Dès le début du traitement avec le rotor-stator sont ajoutées 0,0648 mol d'acide nitrique $HNO_3$, soit 0,20 équivalents par rapport au néodyme, sous la forme d'une solution aqueuse concentrée. Le traitement s'effectue pendant une durée de 30 minutes. Le milieu est ensuite transféré dans un réacteur de 1 litre à double enveloppe muni d'un Dean-Stark, d'un réfrigérant, d'un système d'agitation mécanique à pales, Le mélange est porté au reflux pendant une durée de 210 minutes, puis l'eau du milieu est éliminée par distillation azéotropique. Après sous-tirage du réacteur, on obtient une solution violette translucide et fluide dont les caractéristiques sont présentées dans le tableau 1.

Exemple 4

Le mode opératoire est le même que dans l'exemple 3, sauf que la quantité d'acide nitrique est remplacée par 0,0648 mol d'acide chlorhydrique HCl concentré, soit 0,20 équivalents par rapport au néodyme. On obtient une solution violette translucide et fluide dont les caractéristiques sont présentées dans le tableau 1.

Tableau 1

|  | C.-Ex. 1 | C.-Ex. 2 | C.-Ex.3 | Ex. 1 | Ex. 2 | Ex. 3 | Ex. 4 |
|---|---|---|---|---|---|---|---|
| Stoechiométrie DEHPA/Nd initiale | 3,12 | 3,12 | 3,44 | 3,20 | 3,20 | 3,00 | 3,02 |
| Acide Minéral ou Organique utilisé | - | - | - | HCl | $HNO_3$ | $HNO_3$ | HCl |
| Rapport Molaire Acide / Néodyme | - | - | - | 0,18 | 0,06 | 0,20 | 0,20 |
| Appareillage utilisé pour la dispersion | Non | Non | Non | Trimix | Trimix | Trimix | Trimix |
| Diamètre du mobile (m) | 0.04 | 0.04 | 0.04 | 0.069 | 0.069 | 0.069 | 0.069 |
| Vitesse de rotation (tr/s) | 8.3 | 8.3 | 8.3 | 50 | 50 | 50 | 50 |
| Vitesse périphérique en bout de pale (m.s$^{-1}$) | 1 | 1 | 1 | 10.7 | 10.7 | 10.7 | 10.7 |

(suite)

| | C.-Ex. 1 | C.-Ex. 2 | C.-Ex.3 | Ex. 1 | Ex. 2 | Ex. 3 | Ex. 4 |
|---|---|---|---|---|---|---|---|
| Cisaillement calculé avec k=10 (s$^{-1}$) | 83 | 83 | 83 | 500 | 500 | 500 | 500 |
| Durée de la dispersion en minutes | - | - | - | 20 | 20 | 30 | 30 |
| Durée du reflux en minutes | 300 | 300 | 120 | 0 | 0 | 210 | 240 |
| Durée de la distillation azéo. en minutes | 180 | 180 | 180 | 350 | 350 | 300 | 240 |
| Durée totale de la synthèse en minutes | 480 | 480 | 400 | 370 | 370 | 540 | 510 |
| Aspect du sel | Turbide | Turbide | Turbide | Limpide | Limpide | Limpide | Limpide |
| Présence de solide | oui | oui | oui | Non | Non | Non | Non |
| Taux d'insolubles | nd | nd | 0,3% | 0% | 0% | 0% | 0% |
| % Nd | 2,42% | 2,34% | 2,26% | 2,61% | 2,43% | 7,52% | 7,13% |
| %P | nd | nd | nd | 1,82% | 1,73% | 4,81% | 4,62% |
| Acidité libre en mmol pour 100 g de sel | 5,6 | 5,1 | 11,7 | 5,2 | 4,3 | 11,4 | 9,0 |
| Viscosité en cP | 92 000 | 96 000 | 34 000 | 9000 | 8000 | 2000 | 4000 |
| nd : valeur non déterminée | | | | | | | |

## II PREPARATION DE SYSTEMES CATALYTIQUES

### Synthèse de systèmes catalytiques «témoin» et selon l'invention à partir de ces sels:

En vue de l'obtention de chaque système catalytique, on verse le sel correspondant de néodyme à l'état de gel ou de solution dans un réacteur préalablement nettoyé de ses impuretés. Le gel témoin est préparé selon le brevet US 6,767,927. Les solutions conformes à l'invention sont celles préparées dans les exemples précédents.

- Première étape de « préformation » de chaque système catalytique :

On introduit ensuite du butadiène dans le réacteur à une température de 30°C, ainsi qu'une certaine quantité du solvant hydrocarboné inerte de la synthèse du sel de néodyme.

- Deuxième étape d'alkylation :

On introduit ensuite dans le réacteur de l'hydrure de diisobutylaluminium (HDiBA) à titre d'agent d'alkylation du sel de néodyme, selon une concentration d'environ 1 M. La durée de l'alkylation est de 15 min. Là température de la réaction d'alkylation est égale à 30° C.

- Troisième étape d'halogénation :

On introduit ensuite dans le réacteur du chlorure de diéthylaluminium (CDEA) à titre de donneur d'halogène, selon une concentration d'environ 1 M. La température du milieu est portée à 60° C. La durée de l'halogénation est de 70 minutes.

- Quatrième étape de vieillissement :

On procède ensuite à un vieillissement du mélange ainsi obtenu en maintenant cette température de 60° C pendant une durée de 120 min.

On stocke finalement la solution catalytique sous atmosphère d'azote dans un congélateur, à la température de -15° C.

Les rapports molaires des différents constituants (butadiène, agent d'alkylation et donneur d'halogène) par rapport au sel de néodyme des systèmes catalytiques ainsi préparés sont donnés dans les tableaux qui suivent.

Ces systèmes catalytiques ont ensuite été testés en polymérisation du butadiène conformément aux procédés décrits ci-après.

## III. POLYMERISATION DU BUTADIENE AU MOYEN DES SYSTEMES CATALYTIQUES PREPARES :

Les techniques de caractérisation des polymères sont décrites en Annexe 1.

### 1) Déroulement de la polymérisation:

On utilise, à titre de réacteur de polymérisation, une bouteille "Steinie" de 250 ml préalablement lavée et séchée, et équipée de capsules perforées et d'un septum en caoutchouc. Chaque réaction de polymérisation du butadiène est effectuée sous atmosphère inerte (azote).

Pour chaque polymérisation, on introduit 122 ml de méthylcyclohexane dans ladite bouteille à titre de solvant de polymérisation. On fait barboter à l'azote ce méthylcyclohexane pendant 10 minutes pour éliminer les impuretés.

16 ml (10,4 g) de butadiène fraîchement distillé est ensuite introduit dans la bouteille à l'aide d'une seringue.

Une quantité de 582 $\mu$mol d'HDiBA pour 100 g de butadiène est ensuite introduite dans la bouteille de manière à nettoyer le milieu des impuretés et de manière à jouer le rôle d'agent de transfert pour le contrôle des masses molaires du polymère.

On utilise un rapport massique "solvant de polymérisation (méthylcyclohexane) / monomère (butadiène)" de 9.

Le catalyseur [Nd / Butadiène / HDiBA / CDEA] préparé précédemment est introduit dans la bouteille à hauteur de 192 $\mu$mol de néodyme pour 100 g de butadiène. Il s'agit de la valeur de la base catalytique.

La bouteille est ensuite placée dans un bain thermostaté à la température souhaitée de 90°C.

On utilise 1 ml de méthanol pour stopper les réactions de polymérisation.

On extrait ensuite les polybutadiènes des solutions polymériques ainsi obtenues, soit par stripping à la vapeur et un séchage sur rouleaux à 100°C ou un séchage en étuve à 60°C sous vide avec un léger courant d'azote, soit par dévolatilisation en faisant le vide sous azote à 60° C.

Les paramètres-clés sont la cinétique de polymérisation et la distribution des masses molaires des élastomères obtenus, ainsi que le taux d'unités 1,4-cis. La mesure du taux de conversion du monomère en polymère en fonction du temps de réaction est utilisée pour décrire la cinétique de polymérisation. Ce taux de conversion est déterminé par la technique des extraits secs. La caractérisation de la macrostructure des polymères est réalisée par la méthode SEC. La caractérisation de la microstructure des polymères est réalisée par la méthode "proche infrarouge" (NIR).

Les résultats des essais sont résumés dans le tableau 2 ci-après:

Tableau 2:

| ESSAI | Rapports molaires du système catalytique Nd/Bd/HD/BA/CDEA | Conversion en % (temps en min.) | $\eta_{inh}$ (dl/g) | Mn en g/mol (IP) | Taux 1,4-cis |
|---|---|---|---|---|---|
| Avec le sel de Nd de Ex. 1 | 1 / 30 / 3,5 / 2,9 | 100 (30) | 2,03 | 117 000 (1,77) | 97,0 |
| Avec le sel de Nd de Ex. 2 | 1 / 30 / 3,5 / 2,9 | 100 (30) | 2,10 | 114 000 (1,74) | 97,9 |
| Témoin « 3,5 » | 1 / 30 / 3,5 / 2,9 | 100 (30) | 2,13 | 117000 (1,77) | 98,0 |

Par ailleurs, l'activité catalytique de ces systèmes catalytiques a également été évaluée. Mesurée par la conversion elle est représentée dans le tableau 4 ci-dessous :

Tableau 3:

| Conversion en % | Témoin | Exemple 1 | Exemple 2 |
|---|---|---|---|
| Après 5 mn | 71% | 78% | 69% |
| Après 10 mn | 93% | 95% | 92% |
| Après 15 mn | 97% | 99% | 95% |

Les résultats des essais montrent que les solutions préparées par voie directe selon le procédé de synthèse de l'invention sont utilisables telles quel pour la préparation de catalyseurs efficaces pour la polymérisation stéréospécifique 1,4-cis du butadiène.

Les cinétiques de polymérisations sont équivalentes voir légèrement supérieures à celles observées avec un catalyseur préparé à partir d'un gel commercial témoin. Les distributions de masses moléculaires sont étroites, comme en témoignent les valeurs de l'indice de polydispersité, toutes inférieures à 2. Les polybutadiènes formés présentent un taux de motifs 1,4-cis élevé et supérieur à 97%.

**ANNEXE 1**

**Mesure de la viscosité inhérente**

La viscosité inhérente $\eta$inh est mesurée à 25°C à 0,1 g/dL dans le toluène et caractérise la macrostructure de l'élastomère.

La viscosité est calculée par la formule

$$\eta = \frac{1}{C} \times \ln\left(\frac{T_1}{T_2}\right)$$

avec $\eta$ la viscosité inhérente (dL/g), C la concentration en polymère dans le toluène (g/dL), T1 le temps d'écoulement de la solution de polymère (s) et T2 le temps d'écoulement du toluène (s))

**Caractérisation de la macrostructure par SEC**

a) Principe de la mesure:

La chromatographie d'exclusion par la taille ou SEC (size exclusion chromatography) permet de séparer physiquement les macromolécules suivant leur taille à l'état gonflé sur des colonnes remplies de phase stationnaire poreuse. Les macromolécules sont séparées par leur volume hydrodynamique, les plus volumineuses étant éluées en premier.

Sans être une méthode absolue, la SEC permet d'appréhender la distribution des masses moléculaires d'un polymère. A partir de produits étalons commerciaux, les différentes masses moyennes en nombre (Mn) et en poids (Mw) peuvent être déterminées et l'indice de polydispersité calculé (Ip = Mw/Mn).

b) Préparation du polymère:

Il n'y a pas de traitement particulier de l'échantillon de polymère avant analyse. Celui-ci est simplement solubilisé dans du tétrahydrofuranne à une concentration d'environ 1 g/l.

c) Analyse SEC:

Cas cl) L'appareillage utilisé est un chromatographe « WATERS alliance ». Le solvant d'élution est le tétrahydrofuranne, le débit de 1 ml/min., la température du système de 35° C et la durée d'analyse de 90 min. On utilise un jeu de deux colonnes de dénomination commerciale « STYRAGEL HT6E ».

Le volume injecté de la solution de l'échantillon de polymère est 100 $\mu$l. Le détecteur est un réfractomètre différentiel « WATERS 2140 » et le logiciel d'exploitation des données chromatographiques est le système « WATERS MILLENIUM ».

Cas c2) L'appareillage utilisé est un chromatographe « WATERS, modèle 150C ». Le solvant d'élution est le tétrahydrofuranne, le débit de 0,7 ml/mn, la température du système de 35°C et la durée d'analyse de 90 min. On utilise un jeu de quatre colonnes en série, de dénominations commerciales « SHODEX KS807 », « WATERS type STYRAGEL HMW7» et deux «WATERS STYRAGEL HMW6E ».

Le volume injecté de la solution de l'échantillon de polymère est 100 $\mu$l. Le détecteur est un réfractomètre différentiel « WATERS modèle RI32X » et le logiciel d'exploitation des données chromatographiques est le système « WATERS MILLENIUM » (version 3.00).

**Caractérisation de la microstructure (taux de 1,4-cis) par proche infrarouge (NIR)**

On a utilisé la technique de dosage appelée « proche infrarouge » (NIR). Il s'agit d'une méthode indirecte faisant appel à des élastomères « témoin » dont la microstructure a été mesurée par la technique de RMN13C. On utilise la relation quantitative (loi de Beer-Lambert) existant entre la répartition des monomères dans un élastomère et la forme du spectre NIR de celui-ci. Cette technique est mise en oeuvre en deux étapes :

1) <u>Etalonnage :</u>

On procède à l'acquisition des spectres respectifs des élastomères « témoin ». On établit un modèle mathématique associant une microstructure à un spectre donné, ceci à l'aide de la méthode de régression PLS (Partial Least Squares) reposant sur une analyse factorielle des données spectrales. Les deux documents suivants traitent d'une manière approfondie de la théorie et de la mise en oeuvre de cette méthode d'analyse de données « multi-variées » :

(1) P. GELADI et B. R. KOWALSKI, "Partial Least Squares régression : a tutorial", Analytica Chimica Acta, vol. 185, 1-17 (1986).

(2) M. TENENHAUS, "La régression PLS - Théorie et pratique", Paris, Editions Technip (1998).

2) <u>Mesure :</u>

On procède à un enregistrement du spectre de l'échantillon. Puis on réalise le calcul de la microstructure.

## Revendications

**1.** Procédé de synthèse d'un organophosphate de terre rare **caractérisé en ce qu'**il comprend :

- la dispersion sous fort cisaillement, dans un mélangeur disposant de mobiles d'agitation, d'un oxyde de terre rare dans un milieu biphasique comprenant au moins un solvant organique, de l'eau, un acide organophosphorique et un acide organique ou minéral, et, simultanément ou séquentiellement,
- la réaction de l'oxyde de terre rare avec l'acide organophosphorique et l'acide organique ou minéral,
le milieu étant animé lors de la dispersion d'un taux de cisaillement supérieur à 200 s$^{-1}$ exprimé par la formule

$$\Gamma_{moyen} = k \ N$$

dans laquelle k est une constant allant de 10 à 13 et N est la vitesse des mobiles d'agitation ,

par une vitesse périphérique en bout de pale supérieure à 4 m.s$^{-1}$, exprimée par l'expression :

$$V_{périph} = \pi \ N \ D$$

dans laquelle D est le diamètre des mobiles d'agitation exprimé en mètre et N leur vitesse de rotation exprimée en s$^{-1}$ ,

l'acide organique ou minéral étant choisi parmi l'acide perchlorique $HClO_4$, l'acide chlorique $HClO_3$, l'acide chloreux $HClO_2$, l'acide chlorhydrique $HCl$, l'acide bromique $HBrO_3$, l'acide bromhydrique $HBr$, l'acide iodique $HIO_3$, l'acide periodique $HIO_4$, l'acide orthoperiodique $H_5IO_6$, l'acide iodhydrique $HI$, l'acide trichloroacétique $CCl_3$-COOH, l'acide dichloroacétique $CCl_2H$-COOH, l'acide trifluoroacétique $CF_3COOH$, l'acide nitrique $HNO_3$, l'acide peroxonitrique $HNO_4$, l'acide sulfureux $H_2SO_3$, l'acide sulfurique $H_2SO_4$, les hydrogénosulfates de lithium, de sodium ou de potassium, l'acide fluorosulfurique $FSO_3H$, l'acide sulfonique $HSO_3H$, l'acide trifluoromethanesulfonique $CF_3$-$SO_3H$, l'acide benzène-sulfonique, l'acide naphtalène-sulfonique, l'acide phosphorique $H_3PO_4$, les hydrogénophosphates de lithium, de sodium ou de potassium, l'acide pyrophosphorique $H_4P_2O_7$, l'acide phosphoreux $H_3PO_3$, l'acide hypophosphoreux $H_3PO_2$, l'acide chromique $H_2CrO_4$, l'acide picrique, l'acide maléique, l'acide oxalique et leurs mélanges.

**2.** Procédé de synthèse d'un organophosphate de terre rare selon la revendication 1, **caractérisé en ce que** la vitesse périphérique en bout de pale est comprise entre 10 m.s$^{-1}$ et 50 m.s$^{-1}$.

**3.** Procédé de synthèse d'un organophosphate de terre rare selon la revendication 1 ou 2, **caractérisé en ce que** l'oxyde de terre rare est l'oxyde de néodyme.

**4.** Procédé de synthèse d'un organophosphate de terre rare selon l'une des revendications précédentes, **caractérisé en ce que** l'oxyde de terre rare présente un degré d'hydratation compris entre 0 et 25%.

**5.** Procédé de synthèse d'un organophosphate de terre rare selon l'une des revendications précédentes, **caractérisé en ce que** l'acide organophosphorique est un diester d'acide phosphorique.

**6.** Procédé de synthèse d'un organophosphate de terre rare selon la revendication 5, **caractérisé en ce que** le rapport molaire du diester d'acide phosphorique à la terre rare est de 2,8 à 3,3.

**7.** Procédé de synthèse d'un organophosphate de terre rare selon l'une quelconque des revendications précédentes, **caractérisé en ce que** l'acide minéral est choisi parmi l'acide chlorhydrique HCl et l'acide nitrique $HNO_3$.

**8.** Procédé de synthèse d'un organophosphate de terre rare selon l'une quelconque des revendications précédentes, **caractérisé en ce que** le rapport molaire de l'acide organique ou minéral à la terre rare est compris entre 0 et 0,25.

**9.** Procédé de synthèse d'un organophosphate de terre rare selon l'une quelconque des revendications précédentes, **caractérisé en ce que** le rapport molaire [acide organophosphorique et acide organique ou minéral] par rapport à la terre rare est compris entre 2,7 et 4,25

**10.** Procédé de synthèse d'un organophosphate de terre rare selon l'une quelconque des revendications précédentes, **caractérisé en ce que** le rapport en volume de l'eau par rapport au solvant organique est strictement supérieur à 0 et strictement inférieur à 1.

**11.** Procédé de synthèse d'un organophosphate de terre rare selon l'une quelconque des revendications précédentes, **caractérisé en ce qu'**il se déroule dans sa totalité au sein d'un seul réacteur.

**12.** Procédé de synthèse d'un organophosphate de terre rare selon l'une quelconque des revendications 1 à 10, **caractérisé en ce qu'**il est mis en oeuvre successivement dans deux réacteurs différents, dans un premier réacteur étant adapté pour la dispersion sous fort cisaillement de l'oxyde de terre rare dans le milieu puis dans un deuxième réacteur, dans lequel le mélange réactionnel obtenu dans le premier réacteur est transvasé, pour poursuivre le procédé.

**13.** Procédé de synthèse d'un organophosphate de terre rare selon l'une quelconque des revendications précédentes, **caractérisé en ce que** il comprend, après l'étape de réaction de l'oxyde de terre rare avec l'acide organophosphorique et l'acide organique ou minéral, une étape d'élimination de l'eau par distillation azéotropique.

**14.** Procédé de synthèse d'un organophosphate de terre rare selon l'une quelconque des revendications précédentes, **caractérisé en ce que**, une fois la synthèse de l'organophosphate de terre rare réalisée, le procédé ne comprend pas d'étape subséquente de traitement des effluents.

**15.** Procédé de synthèse d'un organophosphate de terre rare selon l'une quelconque des revendications précédentes, **caractérisé en ce que**, une fois la synthèse de l'organophosphate de terre rare réalisée, les effluents de la réaction contenant éventuellement de l'acide organophosphorique non réagi sont recyclés en amont du procédé

**16.** Procédé de préparation d'un système catalytique "préformé" pour la polymérisation stéréospécifique de diènes conjugués à base d'au moins

- un monomère diène conjugué,
- l'organophosphate de terre rare,
- un agent d'alkylation constitué d'un alkylaluminium de formule $AlR_3$ ou $HAlR_2$ et
- un donneur d'halogène qui appartient à la famille des halogénures d'alkylaluminium à l'exclusion des sesquihalogénures d'alkylaluminium, **caractérisé en ce qu'**il comprend les étapes de préparation d'un organophosphate de terre rare telles que définies dans les revendications 1 à 15.

**Patentansprüche**

1. Verfahren zur Synthese eines Organophosphats einer seltenen Erde, **dadurch gekennzeichnet, dass** es Folgendes aufweist:

    - die Dispersion unter starkem Rühren und Zerkleinern in einer Mischanlage, die über Rührelemente verfügt, eines Oxids seltener Erde in einem Zweiphasensystem, das mindestens ein organisches Lösemittel, Wasser, eine Organophosphorsäure und eine organische oder Mineralsäure aufweist und gleichzeitig oder sequenziell,
    - die Reaktion des Oxids seltener Erde mit der Organophosphorsäure und der organischen oder Mineralsäure, wobei das System bei der Dispersion mit einer Rühr-Zerkleinerungsgeschwindigkeit größer als 200 s$^{-1}$ bewegt wird, ausgedrückt durch die Formel

    $$\Gamma_{mittel} = k\ N$$

    in der k eine Konstante ist, die von 10 bis 13 reicht, und N die Geschwindigkeit der Rührelemente ist,

    durch eine Blattspitzengeschwindigkeit größer als 4 m.s$^{-1}$, ausgedrückt durch den Ausdruck

    $$V_{Umfang} = \pi\ N\ D$$

    in dem D der Durchmesser der Rührelemente ausgedrückt in Meter und N ihre Drehzahl ausgedrückt in s$^{-1}$ ist,

    wobei die organische oder Mineralsäure ausgewählt ist aus Perchlorsäure $HClO_4$, Chlorsäure $HClO_3$, chloriger Säure $HClO_2$, Salzsäure $HCl$, Bromsäure $HBrO_3$, Bromwasserstoffsäure $HBr$, Iodsäure $HIO_3$, Periodsäure $HIO_4$, Orthoperiodsäure $H_5IO_6$, Iodwasserstoffsäure $HI$, Trichloressigsäure $CCl_3\text{-}COOH$, Dichloressigsäure $CCl_2H\text{-}COOH$, Trifluoressigsäure $CF_3COOH$, Salpetersäure $HNO_3$, Peroxonitritsäure $HNO_4$, schwefeliger Säure $H_2SO_3$, Schwefelsäure $H_2SO_4$, den Lithium-, Natrium- oder Kaliumhydrogensulfaten, Fluorschwefelsäure $FSO_3H$, Sulfonsäure $HSO_3H$, Trifluormethansulfonsäure $CF_3\text{-}SO_3H$, Benzolsulfonsäure, Naphthalinsulfonsäure, Phosphorsäure $H_3PO_4$, Lithium-, Natrium- oder Kaliumhydrogenophosphaten, Pyrophosphorsäure $H_4P_2O_7$, phosphoriger Säure $H_3PO_3$, hypophosphoriger Säure $H_3PO_2$, Chromsäure $H_2CrO_4$, Pikrinsäure, Maleinsäure, Oxalsäure und ihren Gemischen.

2. Verfahren zur Synthese eines Organophosphats seltener Erde nach Anspruch 1, **dadurch gekennzeichnet, dass** die Blattspitzengeschwindigkeit zwischen 10 m.s$^{-1}$ und 50 m.s$^{-1}$ liegt.

3. Verfahren zur Synthese eines Organophosphats seltener Erde nach Anspruch 1 oder 2, **dadurch gekennzeichnet, dass** das Oxid seltener Erde das Neodimoxid ist.

4. Verfahren zur Synthese eines Organophosphats seltener Erde nach einem der vorhergehenden Ansprüche, **dadurch gekennzeichnet, dass** das Oxid seltener Erde einen Hydratationsgrad zwischen 0 und 25 % aufweist.

5. Verfahren zur Synthese eines Organophosphats seltener Erde nach einem der vorhergehenden Ansprüche, **dadurch gekennzeichnet, dass** die Organophosphorsäure ein Phosphorsäurediester ist.

6. Verfahren zur Synthese eines Organophosphats seltener Erde nach Anspruch 5, **dadurch gekennzeichnet, dass** das Molverhältnis des Phosphorsäurediesters seltener Erde 2,8 bis 3,3 ist.

7. Verfahren zur Synthese eines Organophosphats seltener Erde nach einem der vorhergehenden Ansprüche, **dadurch gekennzeichnet, dass** die Mineralsäure aus Salzsäure $HCl$ und Salpetersäure $HNO_3$ ausgewählt wird.

8. Verfahren zur Synthese eines Organophosphats seltener Erde nach einem der vorhergehenden Ansprüche, **dadurch gekennzeichnet, dass** das Molverhältnis der organischen oder Mineralsäure zur seltenen Erde zwischen 0 und 0,25 liegt.

9. Verfahren zur Synthese eines Organophosphats seltener Erde nach einem der vorhergehenden Ansprüche, **dadurch gekennzeichnet, dass** das Molverhältnis [Organophosphorsäure und organische oder Mineralsäure] zur seltenen Erde zwischen 2,7 und 4,25 liegt.

10. Verfahren zur Synthese eines Organophosphats seltener Erde nach einem der vorhergehenden Ansprüche, **dadurch gekennzeichnet, dass** das Verhältnis Wasservolumen zu organischem Lösemittel strikt größer als 0 und strikt kleiner als 1 ist.

11. Verfahren zur Synthese eines Organophosphats seltener Erde nach einem der vorhergehenden Ansprüche, **dadurch gekennzeichnet, dass** es vollständig innerhalb eines einzigen Reaktors abgewickelt wird.

12. Verfahren zur Synthese eines Organophosphats seltener Erde nach einem der Ansprüche 1 bis 10, **dadurch gekennzeichnet, dass** es nacheinander in zwei unterschiedlichen Reaktoren ausgeführt wird, in einem ersten Reaktor, der für die Dispersion unter starkem Rühren und Zerkleinern des Oxids seltener Erde in dem System geeignet ist, dann in einem zweiten Reaktor, in den das im ersten Reaktor erzielte Reaktionsgemisch umgefüllt wird, um das Verfahren fortzusetzen.

13. Verfahren zur Synthese eines Organophosphats seltener Erde nach einem der vorhergehenden Ansprüche, **dadurch gekennzeichnet, dass** es nach dem Reaktionsschritt des Oxids seltener Erde mit der Organophosphorsäure und der organischen oder Mineralsäure einen Eliminierungsschritt des Wassers durch azeotrope Destillation aufweist.

14. Verfahren zur Synthese eines Organophosphats seltener Erde nach einem der vorhergehenden Ansprüche, **dadurch gekennzeichnet, dass** nach dem Durchführen der Synthese des Organophosphats seltener Erde das Verfahren keinen darauf folgenden Abwasseraufbereitungsschritt aufweist.

15. Verfahren zur Synthese eines Organophosphats seltener Erde nach einem der vorhergehenden Ansprüche, **dadurch gekennzeichnet, dass** nach dem Ausführen der Synthese des Organophosphats seltener Erde das Abwasser der Reaktion, das eventuell Organophosphorsäure enthält, die nicht reagiert hat, stromaufwärts des Verfahrens recycelt wird.

16. Verfahren zur Vorbereitung eines "vorgebildeten" katalytischen Systems zur stereospezifischen Polymerisation konjugierter Diene auf der Basis von mindestens

    - einem konjugierten Dienmonomer,
    - dem Organophosphat seltener Erde,
    - einem Alkylierungsmittel, das aus einem Alkylaluminium mit der Formel $AlR_3$ oder $HAlR_2$ besteht, und
    - einem Halogendonator, der der Familie der Alkylaluminium-Halogenide mit Ausnahme der Alkylaluminium-Sesquihalogenide angehört, **dadurch gekennzeichnet, dass** es die Vorbereitungsschritte eines Organophosphats seltener Erde, wie sie in den Ansprüchen 1 bis 15 beschrieben sind, aufweist.


**Claims**

1. Process for the synthesis of a rare-earth organophosphate **characterized in that** it comprises:

    - the dispersion, under high shear, in a mixer having agitator impellers, of a rare-earth oxide in a two-phase medium comprising at least one organic solvent, water, an organophosphoric acid and an organic or mineral acid and, simultaneously or sequentially,
    - the reaction of the rare-earth oxide with the organophosphoric acid and the organic or mineral acid,
    the medium being driven, during the dispersion, by a shear rate of greater than 200 s$^{-1}$, expressed by the equation:

$$\Gamma_{\text{average}} = k\ N$$

in which k is a constant ranging from 10 to 13 and N is the speed of the agitator impellers,

by a blade tip peripheral speed of greater than 4 m.s$^{-1}$, expressed by the equation:

$$V_{periph} = \pi \ N \ D$$

in which D is the diameter of the agitator impellers expressed in metres and N is their rotational speed expressed in s$^{-1}$,

the organic or mineral acid being chosen from perchloric acid $HClO_4$, chloric acid $HClO_3$, chlorous acid $HClO_2$, hydrochloric acid HCl, bromic acid $HBrO_3$, hydrobromic acid HBr, iodic acid $HIO_3$, periodic acid $HIO_4$, orthoperiodic acid $H_5IO_6$, hydroiodic acid HI, trichloroacetic acid $CCl_3$-COOH, dichloroacetic acid $CCl_2$H-COOH, trifluoroacetic acid $CF_3COOH$, nitric acid $HNO_3$, peroxonitric acid $HNO_4$, sulphurous acid $H_2SO_3$, sulphuric acid $H_2SO_4$, lithium, sodium or potassium hydrogen sulphates, fluorosulphuric acid $FSO_3H$, sulphonic acid $HSO_3H$, trifluoromethanesulphonic acid $CF_3$-$SO_3H$, benzenesulphonic acid, naphthalenesulphonic acid, phosphoric acid $H_3PO_4$, lithium, sodium or potassium hydrogen phosphates, pyrophosphoric acid $H_4P_2O_7$, phosphorous acid $H_3PO_3$, hypophosphorous acid $H_3PO_2$, chromic acid $H_2CrO_4$, picric acid, maleic acid, oxalic acid and mixtures thereof.

2. Process for the synthesis of a rare-earth organophosphate according to Claim 1, **characterized in that** the blade tip peripheral speed is between 10 m.s$^{-1}$ and 50 m.s1

3. Process for the synthesis of a rare-earth organophosphate according to Claim 1 or 2, **characterized in that** the rare-earth oxide is neodymium oxide.

4. Process for the synthesis of a rare-earth organophosphate according to one of the preceding claims, **characterized in that** the rare-earth oxide has a degree of hydration between 0 and 25%.

5. Process for the synthesis of a rare-earth organophosphate according to any one of the preceding claims, **characterized in that** the organophosphoric acid is a phosphoric acid diester.

6. Process for the synthesis of a rare-earth organophosphate according to Claim 5, **characterized in that** the molar ratio of the phosphoric acid diester to the rare earth is from 2.8 to 3.3.

7. Process for the synthesis of a rare-earth organophosphate according to any one of the preceding claims, **characterized in that** the mineral acid is chosen from hydrochloric acid HCl and nitric acid $HNO_3$.

8. Process for the synthesis of a rare-earth organophosphate according to any one of the preceding claims, **characterized in that** the molar ratio of the organic or mineral acid to the rare earth is between 0 and 0.25.

9. Process for the synthesis of a rare-earth organophosphate according to any one of the preceding claims, **characterized in that** the molar ratio of the [organophosphoric acid and organic or mineral acid] with respect to the rare earth is between 2.7 and 4.25.

10. Process for the synthesis of a rare-earth organophosphate according to any one of the preceding claims, **characterized in that** the volume ratio of water relative to the organic solvent is strictly greater than 0 and strictly less than 1.

11. Process for the synthesis of a rare-earth organophosphate according to any one of the preceding claims, **characterized in that** it takes place, in its entirety, within a single reactor.

12. Process for the synthesis of a rare-earth organophosphate according to any one of Claims 1 to 10, **characterized in that** it is carried out successively in two different reactors, in a first reactor that is suitable for the dispersion, under high shear, of the rare-earth oxide in the medium, then in a second reactor, into which the reaction mixture obtained in the first reactor is decanted, in order to continue the process.

13. Process for the synthesis of a rare-earth organophosphate according to any one of the preceding claims, **characterized in that** it comprises, after the step of reacting the rare-earth oxide with the organophosphoric acid and the organic or mineral acid, a step of removing water by azeotropic distillation.

14. Process for the synthesis of a rare-earth organophosphate according to any one of the preceding claims, **characterized in that**, once the synthesis of the rare-earth organophosphate has been carried out, the process does not comprise a subsequent step of treating the effluents.

15. Process for the synthesis of a rare-earth organophosphate according to any one of the preceding claims, **characterized in that**, once the synthesis of the rare-earth organophosphate has been carried out, the effluents of the reaction possibly containing unreacted organophosphoric acid are recycled upstream of the process.

16. Process for the preparation of a "preformed" catalyst system for the stereospecific polymerization of conjugated dienes based on at least

   - a conjugated diene monomer,
   - the rare-earth organophosphate,
   - an alkylating agent constituted of an alkylaluminium of formula $AlR_3$ or $HAlR_2$ and
   - a halogen donor which belongs to the family of alkylaluminium halides with the exclusion of alkylaluminium sesquihalides,
   **characterized in that** it comprises the steps of preparing a rare-earth organophosphate as defined in claims 1 to 15.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 0238635 A
- WO 0238636 A
- WO 03097708 A
- JP 60023406 B
- EP 1509557 B1
- US 6767927 B1
- US 6111082 A
- US 6482906 B1
- US 5220045 A
- EP 0924214 B1
- WO 2009019100 A
- US 6638918 B
- US 6767927 B

### Littérature non-brevet citée dans la description

- DEHP Complexes of Lanthanides (III) and Actinides (III. *Journal of Alloys and Compounds,* 1994, vol. 213/214, 523-527
- **KOROVIN et al.** *Russian Journal of Inorganic Chemistry,* 1977, vol. 22 (5
- **CHEN TIEN et al.** Préparation and characterization of bis(2-ethylhexyl) phosphate(P204) rare earth. *K'o Hsueh T'ung Pao,* 1981, vol. 26 (13), 794-6
- **P. GELADI ; B. R. KOWALSKI.** Partial Least Squares régression : a tutorial. *Analytica Chimica Acta,* 1986, vol. 185, 1-17
- **M. TENENHAUS.** La régression PLS - Théorie et pratique. 1998